# FASCICULE DE BREVET EUROPEEN

(11) **EP 0 878 186 B1**
(45) Date de publication et mention de la délivrance du brevet: **29.12.1999**
(21) Numéro de dépôt: 98400602.3
(22) Date de dépôt: 13.03.1998
(51) Int. Cl.: A61K 7/06, A61K 7/48, A61K 7/50

(54) **Compositions cosmétiques détergentes et utilisation**
Kosmetische Tensid Zusammensetzung sowie ihre Verwendung
Cosmetic detergent compositions and their use

(30) Priorité: 07.04.1997 FR 9704220
(43) Date de publication de la demande: 18.11.1998
(73) Titulaire: L'OREAL, 75008 Paris (FR)
(72) Inventeur: Decoster, Sandrine, 93800 Epinay sur Seine (FR); Beauquey, Bernard, 92110 Clichy (FR)
(74) Mandataire: Le Blainvaux Bellegarde, Françoise

(56) Documents cités:
- EP-A- 0 392 320
- EP-A- 0 521 748
- WO-A-93/08787
- WO-A-94/06403
- WO-A-95/01152
- WO-A-96/32919

## Description

La présente invention concerne de nouvelles compositions cosmétiques à propriétés améliorées destinées simultanément au nettoyage et au conditionnement des matières kératiniques telles que les cheveux ou la peau, et comprenant, dans un support cosmétiquement acceptable, une base lavante constituée de tensioactifs à pouvoir détergent alkyléthersulfate dans laquelle sont également présents à titre d'agents conditionneurs, des polymères cationiques choisis parmi les homopolymères de la famille des diallyldiméthylammonium en association avec des silicones non aminées. L'invention concerne aussi l'utilisation desdites compositions dans l'application cosmétique susmentionnée.

Pour le nettoyage et/ou le lavage des cheveux et/ou de la peau, l'utilisation de compositions détergentes (shampooing ou gel-douche) à base essentiellement d'agents tensioactifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes. En outre, sur des cheveux sensibilisés, il est connu d'utiliser de préférence un mélange de silicone et de polymère cationique.

Toutefois, et malgré les progrès réalisés récemment dans le domaine des shampooings à base de polymères cationiques et de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

Ainsi, à la suite d'importantes recherches menées sur la question, il a maintenant été trouvé par la Demanderesse, de façon totalement inattendue et surprenante, qu'en utilisant une silicone particulière et convenablement sélectionnée, telles que définie ci-après, dans des compositions détergentes contenant des polymères cationiques particuliers à titre d'agents conditionneurs, il est possible d'améliorer de manière substantielle et significative les propriétés cosmétiques attachées à ces dernières, et ceci tout en conservant leur bon pouvoir lavant intrinsèque.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse.

Ainsi, selon la présente invention, il est maintenant proposé de nouvelles compositions détergentes et conditionnantes comprenant, dans un milieu cosmétiquement acceptable, (A) une base lavante comprenant au moins un tensioactif anionique de type alkyléthersulfate et (B) un système conditionneur comprenant au moins une silicone insoluble non aminée et au moins un polymère cationique choisi parmi les homopolymères comportant comme constituant principal de la chaîne des motifs répondant aux formules (I) ou (I'): 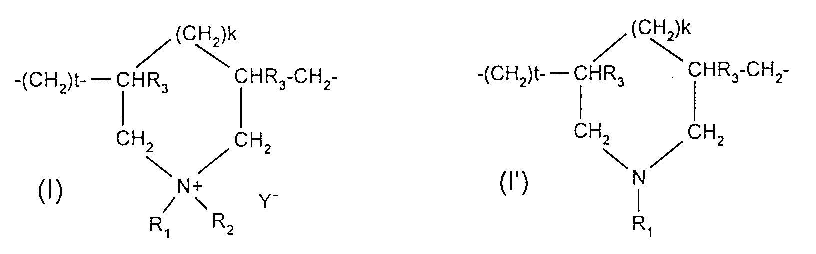 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

L'invention a également pour objet l'utilisation en cosmétique des compositions ci-dessus pour le nettoyage et/ou le conditionnement des matières kératiniques telles que les cheveux et la peau.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

### A- BASE LAVANTE :

Les compositions conformes à l'invention comprennent nécessairement une base lavante, généralement aqueuse, comprenant au moins un ou plusieurs tensioactifs anioniques de type alkyl éther sulfate.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 2 % à 50 % en poids, de préférence de 10 % à 35 % en poids, et encore plus préférentiellement de 12 % à 25 % en poids, du poids total de la composition finale.

Les tensioactifs anioniques de type alkyl éthersulfate utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkyléthersulfates, alkylamidoéthersulfates, alkylaryléthersulfates; les alkyléthersulfosuccinates, le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène pouvant aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent notamment de 2 à 5 groupements d'oxyde d'éthylène.

Les tensioactifs anioniques sont généralement présents à raison de 1 à 50 % en poids, de préférence de 5 à 20 % en poids, par rapport au poids total de la composition.

Dans les compositions conformes à l'invention, on utilise de préférence des mélanges de tensioactifs, en particulier des mélanges d'agents tensioactifs anioniques ou des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alphadiols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Les tensioactifs non ioniques ou amphotères sont généralement présents à raison de 1 à 50 % en poids, de préférence de 2 à 20 % en poids, par rapport au poids total de la composition.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs détergents préférés pour la mise en oeuvre de la présente invention.

On utilise de préférence un agent tensioactif anionique choisi parmi les alkyl (C₁₂-C₁₄)éthersulfates de sodium, de triéthanolamine ou d'ammonium oxyéthylénés à environ 2,2 moles d'oxyde d'éthylène, et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocoylbétaïne commercialisée sous la dénomination "DEHYTON AB 30" en solution aqueuse à 32 % de MA par la société HENKEL.

### B) SYSTEME CONDITIONNEUR

### (i) Polymère(s) cationique(s) :

Au sens de la présente invention, l'expression "polymère cationique" désigne tout polymère contenant des groupements cationiques et/ou des groupements ionisables en groupements cationiques.

Les compositions conformes à l'invention comprennent en outre nécessairement un polymère cationique choisi les homopolymères comportant comme constituant principal de la chaîne des motifs répondant aux formules (I) et/ou (I'): 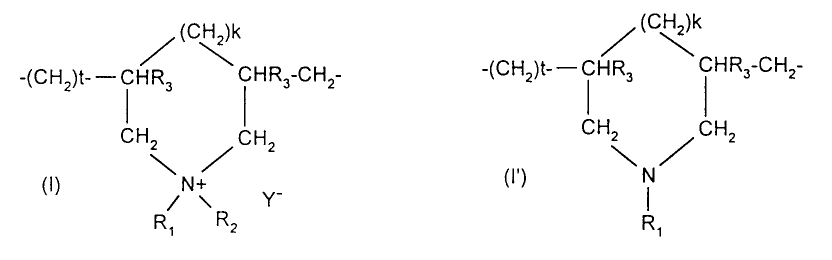 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759, dans son certificat d'addition 2.190.406 ou dans les brevets US3,996,146 et US3,288,770.

De préférence , R₁ et R₂ désignent indépendamment l'un de l'autre, méthyle ou éthyle et R₃ désigne un atome d'hydrogène.

Les polymères cationiques utilisés ont généralement une masse moléculaire moyenne en poids comprise entre 500 et 5.10⁶ environ, et de préférence comprise entre 10³ et 5.10⁵ environ.

De préférence, les polymères cationiques selon l'invention ont une densité de charge cationique supérieure ou égale à environ 5 meq./g et plus particulièrement de 5 à 10 meq./g. La charge cationique représente le nombre de groupement cationique par gramme de polymère.

Parmi les polymères définis ci-dessus, on peut citer plus particulièrement les homopolymères de chlorure de diallyldiméthylammonium tel que celui commercialisé sous la dénomination "MERQUAT® 100" par la société CALGON.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

### (ii)- Silicones insolubles

Selon une caractéristique essentielle des compositions détergentes conformes à l'invention, ces dernières contiennent en outre au moins une silicone insoluble non aminée.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone non aminée toute silicone ne comportant pas au moins une amine primaire, secondaire, tertiaire ou un groupement ammonium quaternaire.

Les silicones utilisables conformément à l'invention sont en particulier des polyorganosiloxanes pouvant se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE® 70045 V 2" par RHONE POULENC, le décaméthylcyclopentasiloxane commercialisé sous le nom de "VOLATILE SILICONE 7158" par UNION CARBIDE, "SILBIONE® 70045 V 5" par RHONE POULENC, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 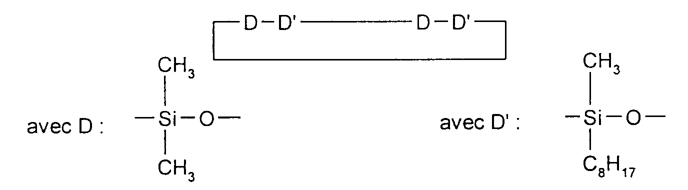
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200®" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".
   On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles SILBIONE® des séries 47 et 70 047 ou les huiles MIRASIL commercialisées par RHONE POULENC telles que par exemple l'huile 70 047 V 500 000 ;
- les huiles de la série MIRASIL® commercialisées par la société RHONE POULENC ;
- les huiles de la série 200 de la société DOW CORNING ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL® WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE® de la série 70 641 de RHONE POULENC ;
. les huiles des séries RHODORSIL 70 633 et 763 de RHONE POULENC ;
. l'huile DOW CORNING 556® COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des poids moléculaires moyens en poids élevés compris entre 200 000 et 3 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C12) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 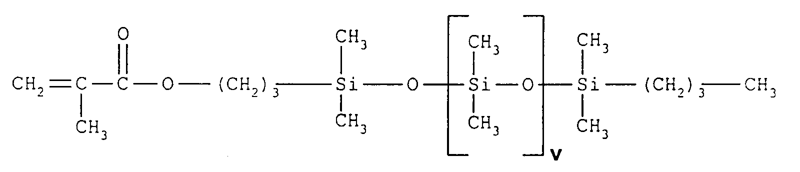 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acryiique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont:
- les silicones non volatiles choisies dans la famille des polyalkylsiloxanes à groupements terminaux triméthylsilyle telles que les huiles polydiméthylsiloxane ayant une viscosité comprise entre 0,2 et 2,5 m²/s à 25° C des séries SILBIONE 70047 et 47 et plus particulièrement l'huile 70 047 V 500 000 commercialisées par la société RHONE POULENC, les polyalkylsiloxanes à groupements terminaux diméthylsilanol ou les polyalkylarylsiloxanes tels que l'huile SILBIONE 70641 V 200 commercialisée par la société RHONE POULENC ;

Les compositions cosmétiques conformes à l'invention renferment les silicones non aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, I'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses telles que le distéaryléther ou le 1-hexadécyloxy octadodécanol.

Les compositions conformes à l'invention peuvent éventuellement contenir en outre d'autres agents ayant pour effet d'améliorer les propriétés cosmétiques des matières kératiniques. On peut citer à ce sujet les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les filtres hydro ou liposolubles, et leurs mélanges.

Bien entendu, l'homme du métier veillera à choisir ce ou ces éventuels composés complémentaires et/ou leurs quantités de manière telle que les propriétés avantageuses attachées intrinsèquement à l'association (base lavante + polymère cationique spécifique + silicone non aminée) conforme à l'invention ne soient pas, ou substantiellement pas, altérées par la ou les adjonctions envisagées.

Ces compositions peuvent se présenter sous la forme de liquides plus ou moins épaisses, de crèmes ou de gel et elles conviennent principalement au lavage, au soin des matières kératiniques telles que la peau ou les cheveux.

Les compositions selon l'invention sont utilisés de préférence comme shampooings pour le lavage et le conditionnement des cheveux et ils sont appliqués dans ce cas-là sur le cheveux humides dans des quantités efficaces pour les laver, cette application étant suivie d'un rinçage à l'eau.

Les compositions conformes à l'invention sont également utilisables comme gels douche pour le lavage et le conditionnement des cheveux et de la peau, auquel cas ils sont appliqués sur la peau et les cheveux humides et sont rincés après application.

L'invention a également pour objet l'utilisation en cosmétique des compositions ci-dessus pour le nettoyage, le conditionnement des matières kératiniques telles que les cheveux et la peau.

Les compositions peuvent par exemple être utilisées pour le démaquillage des matières kératiniques telles que la peau (par exemple du visage, du cou ou des lèvres), les cils, les sourcils.

L'invention a également pour objet un procédé de lavage et de conditionnement des matières kératiniques telles que les cheveux consistant à appliquer sur lesdites matières mouillées une quantité efficace d'une composition telle que définie ci-dessus, puis à effectuer un rinçage à l'eau après un éventuel temps de pause.

Des exemples concrets, mais nullement limitatifs, illustrant l'invention vont maintenant être donnés.

### EXEMPLE 1

On a réalisé deux compositions de shampooing, l'une conforme à l'invention (composition A) et l'autre comparative (composition B) :

| | A Invention | B Comparatif |
|---|---|---|
| - Lauryléthersulfate de sodium (C₁₂/C₁₄ à 70/30) à 2,2 moles d'oxyde d'éthylène en solution aqueuse à 70% de MA (MA = matière active) | 15,5 gMA | 15,5 gMA |
| - Cocobétaïne à 32% MA(*) | 2,9 gMA | 2,9 gMA |
| - Polymère cationique (**) | 0,6 g | - |
| - Polymère cationique (****) | - | 0,6 g |
| - Silicone insoluble non aminée (***) | 2 g | 2 g |
| - Cétostéarylsulfate de sodium | 0,75 g | 0,75 g |
| - Distéarate d'éthylèneglycol | 2 g | 2 g |
| - Acide citrique qs pH | 5 | 5 |
| - Parfum, Conservateurs | qs | qs |
| - Eau déminéralisée qs | 100 g | 100 g |

| | | |
|---|---|---|
| (*) DEHYTON AB 30 de HENKEL | | |
| (**) : Homopolymère de chlorure de diméthyl diallyl ammonium commercialisée sous la dénomination MERQUAT 100 (PM 400.000) par la société CALGON | | |
| (***) : Polydiméthylsiloxane commercialisé sous la dénomination FLUID DC 200 de viscosité 60.000 Cst. par la société DOW CORNING | | |
| (****) : Cellulose quaternisée commercialisée sous la dénomination JR 400 par la société UNION CARBIDE | | |

On effectue un shampooing en appliquant environ 12 g de la composition A sur des cheveux préalablement mouillés. On fait mousser le shampooing puis on rince abondamment à l'eau.
On procède selon le même mode opératoire que ci-dessus avec la composition comparative B.

Un panel d'experts évalue le démêlage des cheveux mouillés, le démêlage des cheveux humides séchés, la douceur et le lissage des cheveux séchés.

Tous les experts indiquent une amélioration nette de ces propriétés pour les cheveux traités avec la composition A selon l'invention.

### EXEMPLE 2

On a réalisé la composition de shampooing suivante :

| | |
|---|---|
| - Lauryléthersulfate de sodium (C₁₂/C₁₄ à 70/30) à 2,2 moles d'oxyde d'éthylène en solution aqueuse à 70% de MA (MA = matière active) | 14 gMA |
| - Cocobétaïne à 30% MA(*) | 1,8 gMA |
| - Polymère cationique (**) | 0,6 gMA |
| - Silicone insoluble non aminée (***) | 3 g MA |
| - Mélange d'alcool cétylique et de 1-(hexadécyloxy)-2-octadécanol | 2,5 g |
| - Monoisopropanolamide d'acides de coprah | 1 g |
| - Acide citrique qs pH | 6 |
| - Parfum, Conservateurs | qs |
| - Eau déminéralisée qsp | 100 g |

| | |
|---|---|
| (*) : DEHYTON AB 30 de HENKEL | |
| (**) : Homopolymère de chlorure de diméthyl diallyl ammonium en solution aqueuse à 40% de matière active commercialisé sous la dénomination MERQUAT 100 (PM 400.000) par la société CALGON | |
| (***) : Polydiméthylsiloxane en émulsion aqueuse à 50% de matière active commercialisé sous la dénomination DC 2-1691 par la société DOW CORNING | |

### EXEMPLE 3

On a réalisé la composition de shampooing suivante :

| | |
|---|---|
| - Lauryléthersulfate de sodium (C₁₂/C₁₄ 70/30 en poids) à 2,2 moles d'oxyde d'éthylène en solution aqueuse à 70% de MA (MA = matière active) | 14 gMA |
| - Cocoamidoéthyl (N-hydroxyéthyl, N-carboxyméthyl)glycinate de sodium (*) | 1,8 gMA |
| - Polymère cationique (**) | 0,6 gMA |
| - Silicone insoluble non aminée (***) | 3 g MA |
| - Mélange d'alcool cétylique et de 1 -(hexadécyloxy)-2-octadécanol | 2,5 g |
| - Monoisopropanolamide d'acides de coprah | 1 g |
| - Acide citrique qs pH | 7 |
| - Parfum, Conservateurs | qs |
| - Eau déminéralisée qsp | 100 g |

| | |
|---|---|
| (*) : CHIMEXANE HD de CHIMEX | |
| (**) : Homopolymère de chlorure de diméthyl diallyl ammonium commercialisé sous la dénomination MERQUAT 100 (PM 400.000) par la société CALGON en solution aqueuse à 40% de matière active | |
| (***) : Polydiméthylsiloxane (viscosité 300 000 Cst) commercialisé sous la dénomination SILICONE FLUID AK 300.000 par la société WACKER | |

## Revendications

1. Composition détergente et conditionnante, caractérisée par le fait qu'elle comprend, dans un milieu cosmétiquement acceptable, (A) une base lavante comprenant au moins un tensioactif anionique de type alkyléthersulfate et (B) un système conditionneur comprenant au moins une silicone insoluble non aminée et au moins un polymère cationique choisi parmi les homopolymères comportant comme constituant principal de la chaîne des motifs répondant aux formules (I) et/ou (I'): 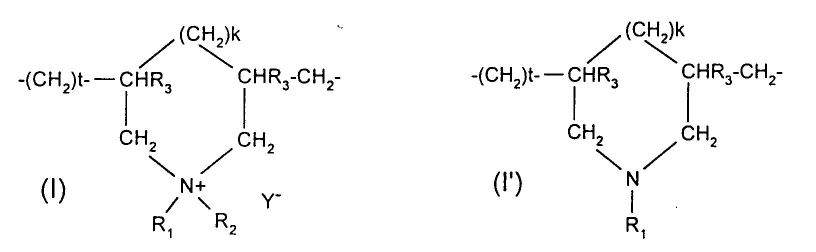 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

2. Composition selon la revendication précédente, caractérisée en ce que les tensioactifs anioniques de type alkyléthersulfate sont les sels des alkyléther sulfates, alkylamidoéther sulfates, alkylaryléther sulfates; alkyléther sulfosuccinates, le radical alkyle ou acyle de tous ces différents composés comportant de préférence de 8 à 24 atomes de carbone, et le radical aryle désignant de préférence un groupement phényle ou benzyle.

3. Composition selon l'une quelconque des revendications précédentes, caractérisée en ce que le tensioactif anionique de type alkyléthersulfate est choisi parmi les sels d'alkyléthersulfates en C₈-C₁₄ et plus particulièrement ceux en C₁₂-C₁₄.

4. Composition selon l'une quelconque des revendications précédentes, caractérisée par le fait que R₁ et R₂, indépendamment l'un de l'autre, désignent méthyle ou éthyle et R₃ désigne un atome d'hydrogène.

5. Composition selon l'une quelconque des revendications précédentes, caractérisée par le fait que ledit polymère cationique est choisi parmi les homopolymères du chlorure de diallyldiméthylammonium.

6. Composition selon l'une quelconque des revendications 1 à 5, caractérisée par le fait que les silicones insolubles et non aminées sont choisies parmi les polyorganosiloxanes se présentant sous forme d'huiles, de cires, de résines ou de gommes.

7. Composition selon l'une quelconque des revendications précédentes, caractérisée par le fait que ladite base lavante est présente à une teneur pondérale comprise entre 2 % et 50 % par rapport au poids total de la composition, de préférence entre 10 % et 35 % en poids et plus particulièrement entre 12 % à 25 % en poids.

8. Composition selon l'une quelconque des revendications précédentes, caractérisée par le fait que le ou lesdits tensioactifs anioniques sont présents à raison de 1 à 50 % en poids, de préférence de 5 à 20 % en poids, par rapport au poids total de la composition.

9. Composition selon l'une quelconque des revendications précédentes, caractérisée par le fait que ledit polymère cationique est présent à une teneur pondérale comprise entre 0,001 % et 10 % par rapport au poids total de la composition, de préférence entre 0,005 % et 5 % en poids et plus particulièrement entre 0,01 % et 3 % en poids.

10. Composition selon l'une quelconque des revendications précédentes, caractérisée par le fait que ladite silicone insoluble non aminée est présente à une teneur pondérale comprise entre 0,05 et 10% par rapport au poids total de la composition, de préférence entre 0,1 et 5 % en poids.

11. Utilisation d'une composition telle définie à l'une quelconque des revendications précédentes pour le nettoyage et/ou le conditionnement des matières kératiniques.

12. Procédé de lavage et de conditionnement des matières kératiniques telles que les cheveux consistant à appliquer sur lesdites matières mouillées une quantité efficace d'une composition telle que définie à l'une quelconque des revendications 1 à 10, puis à effectuer un rinçage à l'eau après un éventuel temps de pause.

## Claims

1. Detergent and conditioning composition, characterized in that it comprises, in a cosmetically acceptable medium, (A) a washing base comprising at least one anionic surfactant of alkyl ether sulphate type and (B) a conditioning system comprising at least one insoluble non-amino silicone and at least one cationic polymer chosen from homopolymers containing, as main constituents of the chain, units corresponding to formulae (I) and/or (I'): 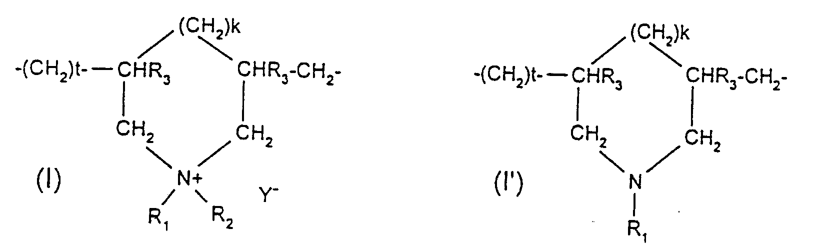 in which formulae k and t are equal to 0 or 1, the sum k + t being equal to 1; R₃ denotes a hydrogen atom or a methyl radical; R₁ and R₂, independently of each other, denote an alkyl group having from 1 to 22 carbon atoms, a hydroxyalkyl group in which the alkyl group preferably has 1 to 5 carbon atoms, a lower (1 to 5 carbon atoms) amido alkyl group, or R₁ and R₂ can denote, together with the nitrogen atom to which they are attached, heterocyclic groups such as piperidyl or morpholinyl; Y⁻ is an anion such as bromide, chloride, acetate, borate, citrate, tartrate, bisulphate, bisulphite, sulphate or phosphate.

2. Composition according to the preceding claim, characterized in that the anionic surfactants of alkyl ether sulphate type are salts of alkyl ether sulphates, alkylamido ether sulphates, alkylaryl ether sulphates or alkyl ether sulphosuccinates, the alkyl or acyl radical of all these various compounds preferably containing from 8 to 24 carbon atoms, and the aryl radical preferably denoting a phenyl or benzyl group.

3. Composition according to either of the preceding claims, characterized in that the anionic surfactant of alkyl ether sulphate type is chosen from C₈-C₁₄ and more particularly C₁₂-C₁₄ alkyl ether sulphate salts.

4. Composition according to any one of the preceding claims, characterized in that R₁ and R₂, independently of each other, denote methyl or ethyl and R₃ denotes a hydrogen atom.

5. Composition according to any one of the preceding claims, characterized in that the said cationic polymer is chosen from diallyldimethylammonium chloride homopolymers.

6. Composition according to any one of Claims 1 to 5, characterized in that the insoluble non-amino silicones are chosen from polyorganosiloxanes in the form of oils, waxes, resins or gums.

7. Composition according to any one of the preceding claims, characterized in that the said washing base is present in a weight content of between 2% and 50% relative to the total weight of the composition, preferably between 10% and 35% by weight and more particularly between 12% and 25% by weight.

8. Composition according to any one of the preceding claims, characterized in that the said anionic surfactant(s) is(are) present in a proportion of from 1 to 50% by weight, preferably from 5 to 20% by weight, relative to the total weight of the composition.

9. Composition according to any one of the preceding claims, characterized in that the said cationic polymer is present in a weight content of between 0.001% and 10% relative to the total weight of the composition, preferably between 0.005% and 5% by weight and more particularly between 0.01% and 3% by weight.

10. Composition according to any one of the preceding claims, characterized in that the said insoluble non-amino silicone is present in a weight content of between 0.05 and 10% relative to the total weight of the composition, preferably between 0.1 and 5% by weight.

11. Use of a composition as defined in any one of the preceding claims, for cleansing and/or conditioning keratin substances.

12. Process for washing and conditioning keratin substances such as the hair, which consists in applying an effective amount of a composition as defined in any one of Claims 1 to 10 to the said wet substances, and then in rinsing with water, after optionally leaving the composition on the keratin substances for a while.

## Patentansprüche

1. Reinigende und konditionierende Zusammensetzung,
dadurch gekennzeichnet, daß
sie in einem kosmetisch akzeptablen Medium (A) eine reinigende Basisformulierung, die mindestens einen anionischen grenzflächenaktiven Stoff vom Alkylethersulfat-Typ enthält, und (B) ein Konditioniersystem enthält, das mindestens ein nicht aminiertes, unlösliches Silicon und mindestens ein kationisches Polymer enthält, das unter den Homopolymeren ausgewählt ist, die als Hauptbestandteile der Kette Einheiten der Formel (I) und/oder (I') enthalten: 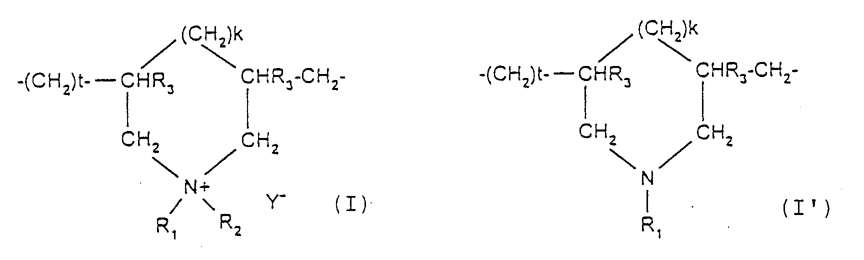 worin k und t 0 oder 1 bedeuten, wobei die Summe k + t 1 ist; R₃ ein Wasserstoffatom oder eine Methylgruppe bedeutet; R₁ und R₂ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine niedere Amidoalkylgruppe (1 bis 5 Kohlenstoffatome) bedeuten oder R₁ und R₂ gemeinsam mit dem Stickstoffatom, an das sie gebunden sind, eine heterocyclische Gruppe bilden können, beispielsweise Piperidinyl oder Morpholinyl; und Y⁻ ein Anion bedeutet, beispielsweise Bromid, Chlorid, Acetat, Borat, Citrat, Tartrat, Bisulfat, Bisulfit, Sulfat, Phosphat.

2. Zusammensetzung nach dem vorhergehenden Anspruch, dadurch gekennzeichnet, daß die anionischen grenzflächenaktiven Stoffe vom Alkylethersulfat-Typ Salze von Alkylethersulfaten, Alkylamidoethersulfaten und Alkylarylethersulfaten oder Alkylethersulfosuccinate sind, wobei die Alkyl- oder Acylgruppe der verschiedenen Verbindungen vorzugsweise 8 bis 24 Kohlenstoffatome aufweist und die Arylgruppe vorzugsweise eine Phenyl- oder Benzylgruppe bedeutet.

3. Zusammensetzung nach einem der vorhergehenden Ansprüche, dadurch gekennzeichnet, daß der anionische grenzflächenaktive Stoff vom Alkylethersulfat-Typ unter den Salzen von Alkylethersulfaten mit 8 bis 14 Kohlenstoffatomen und insbesondere 12 bis 14 Kohlenstoffatomen ausgewählt ist.

4. Zusammensetzung nach einem der vorhergehenden Ansprüche, dadurch gekennzeichnet, daß die Gruppen R₁ und R₂ unabhängig voneinander Methyl oder Ethyl bedeuten und R₃ Wasserstoff bedeutet.

5. Zusammensetzung nach einem der vorhergehenden Ansprüche, dadurch gekennzeichnet, daß das kationische Polymer unter den Homopolymeren von Diallyldimethylammoniumchlorid ausgewählt ist.

6. Zusammensetzung nach einem der Ansprüche 1 bis 5, dadurch gekennzeichnet, daß die unlöslichen und nicht aminierten Silicone unter den Polyorganosiloxanen ausgewählt sind, die in Form von Ölen, Wachsen, Harzen oder Gummis vorliegen.

7. Zusammensetzung nach einem der vorhergehenden Ansprüche, dadurch gekennzeichnet, daß die reinigende Basisformulierung in einem Gewichtsanteil im Bereich von 2 bis 50 %, bezogen auf das Gesamtgewicht der Zusammensetzung, vorzugsweise im Bereich von 10 bis 35 Gew.-% und insbesondere im Bereich von 12 bis 25 Gew.-% vorliegt.

8. Zusammensetzung nach einem der vorhergehenden Ansprüche, dadurch gekennzeichnet, daß der anionische grenzflächenaktive Stoff oder die anionischen grenzflächenaktiven Stoffe in einem Mengenanteil von 1 bis 50 Gew.-% und vorzugsweise 5 bis 20 Gew.-%, bezogen auf das Gesamtgewicht der Zusammensetzung, vorliegen.

9. Zusammensetzung nach einem der vorhergehenden Ansprüche, dadurch gekennzeichnet, daß das kationische Polymer in einem Gewichtsanteil von 0,001 bis 10 Gew.-%, bezogen auf das Gesamtgewicht der Zusammensetzung, vorzugsweise im Bereich von 0,005 bis 5 Gew.-% und insbesondere im Bereich von 0,01 bis 3 Gew.-% vorliegt.

10. Zusammensetzung nach einem der vorhergehenden Ansprüche, dadurch gekennzeichnet, daß das nicht aminierte unlösliche Silicon in einem Gewichtsanteil im Bereich von 0,05 bis 10 Gew.-%, bezogen auf das Gesamtgewicht der Zusammensetzung, und vorzugsweise im Bereich von 0,1 bis 5 Gew.-% vorliegt.

11. Verwendung einer Zusammensetzung nach einem der vorhergehenden Ansprüche zur Reinigung und/oder Konditionierung von Keratinmaterialien.

12. Verfahren zum Waschen und Konditionieren von Keratinmaterialien, wie dem Haar, das darin besteht, auf die feuchten Materialien eine wirksame Menge einer Zusammensetzung nach einem der Ansprüche 1 bis 10 aufzutragen und anschließend gegebenenfalls nach einer Einwirkzeit mit Wasser zu spülen.
